# EUROPÄISCHE PATENTSCHRIFT

(11) **EP 1 098 868 B1**
(45) Veröffentlichungstag und Bekanntmachung des Hinweises auf die Patenterteilung: **25.02.2004**
(21) Anmeldenummer: 99934646.3
(22) Anmeldetag: 09.07.1999
(51) Int. Cl.: C07C 67/26, C07C 69/24, C07C 69/52, C08G 65/26

(54) **VERFAHREN ZUR HERSTELLUNG ALKOXYLIERTER CARBONSÄUREESTER**
METHOD FOR PREPARING ALCOXYLATED CARBOXYLIC ACID ESTERS
PROCEDE POUR LA PREPARATION D'ESTERS D'ACIDE CARBOXYLIQUE ALCOXYLES

(30) Priorität: 18.07.1998 DE 19832427
(43) Veröffentlichungstag der Anmeldung: 16.05.2001
(73) Patentinhaber: Cognis Deutschland GmbH & Co. KG, 40589 Düsseldorf (DE)
(72) Erfinder: BEHLER, Ansgar, D-46240 Bottrop (DE); FOLGE, Almud, D-40764 Langenfeld (DE)
(86) Internationale Anmeldenummer: PCT/EP1999/004836
(87) Internationale Veröffentlichungsnummer: WO 2000/003969

(56) Entgegenhaltungen:
- EP-A- 0 335 295
- DE-C- 19 611 508

## Beschreibung

Die Erfindung betrifft ein Verfahren zur Herstellung alkoxylierter Carbonsäureester in Gegenwart einer basischen, homogenen Katalysatormischung enthaltend Natrium- und Kalium- Verbindungen in einem ausgewählten Mischungsverhältnis.

Alkoxylierte Carbonsäureester, vorzugsweise ethoxylierte Carbonsäuremethylester, die auch als Methylesterethoxylate bezeichnet werden, stellen bekannte nichtionische Tenside dar, die wegen ihrer ausgezeichneten Waschleistung in letzter Zeit erheblich an Interesse gewonnen haben.

Zu ihrer Herstellung setzt man gewöhnlich Carbonsäureester mit Alkylenoxiden in Gegenwart basischer Katalysatoren um, wobei die Alkylenoxide in die Carbonylesterbindung eingelagert (Insertion) werden. Die Insertion von Alkylenoxiden in die Carbonylesterbindung ist wesentlich schwieriger als die Anlagerung von Alkylenoxiden an Verbindungen mit aciden Wasserstoffatomen und gelingt daher nur unter Einsatz besonderer Katalysatoren.

Aus den beiden Patentschriften **EP-B1- 0 339 425** und **EP-B1- 0 523 089** ist die Verwendung von calcinierten bzw. mit Fettsäuren modifizierten Hydrotalciten für die Ethoxylierung von Fettsäureestern bekannt. In der Offenlegungsschrift **DE-A1 44 46 064** wird vorgeschlagen, die Ethoxylierung von Methylestem in Gegenwart von Mischmetalloxiden durchzuführen, deren Oberfläche mit Metallhydroxiden bzw. Metallalkoxiden modifiziert worden ist. Diese Verfahren weisen jedoch eine Reihe von Nachteilen auf: Der Einsatz heterogener, d.h. im Reaktionsgemisch nicht löslicher Katalysatoren ist technisch aufwendiger, da der Feststoff nicht wie eine Flüssigkeit über eine automatische Dosiervorrichtung eingebracht werden kann, sondern üblicherweise von Hand in der Reaktor geschaufelt werden muß. Auch die Abtrennung bereitet Probleme, da der Katalysator in der Regel so feinteilig ist, daß die Filtration nur über spezielle Filterkerzen gelingt. Ein Verbleiben des Katalysators im Reaktionsendprodukt ist indes auch nicht möglich, da es sonst zu Austrübungen und zu Sedimentation kommen kann.

Aus der Patentanmeldung **EP-A2- 0335295** ist ein Verfahren zur Herstellung von alkoxylierten Carbonsäureestern unter homogener Katalyse bekannt, das die oben geschilderten Nachteile der heterogenen Katalyse nicht aufweist. Als Katalysatoren werden Alkalimetall- oder Erdalkalimetall- Verbindungen aus der Gruppe der Hydroxide, Oxide und Alkoholate beschrieben, die jedoch stets einzeln und nicht in Mischung eingesetzt werden. Derartige Katalysatoren alleine beschleunigen die Reaktion jedoch nicht im gewünschten Maße, so daß lange Reaktionszeiten in Kauf genommen werden müssen.

In den beiden Schriften **DE- C- 196 11508** sowie **EP- A1- 0783012** wird vorgeschlagen, zu den Alkali- und Erdalkaliverbindungen als Co-Katalysator Alkylenglykole bzw. Siliciumverbindungen zuzugeben. Bei letzterem treten jedoch aufgrund der Unlöslichkeit des Co-Katalysators im Reaktionsgemisch die gleichen Nachteile auf wie bei der heterogenen Katalyse. Bei der Variante mit den Alkylenglykolen liegt zwar weiterhin eine homogene Katalyse vor, aber da die Alkylenglykole auch mit den eingesetzten Alkylenoxiden während des Verfahrens reagieren, entstehen Polyethylenglykole, die aufgrund ihrer Neigung zu Sedimentationen unerwünscht sind.

Die Aufgabe der Erfindung hat somit darin bestanden, ein Verfahren zur Herstellung von alkoxylierten Carbonsäureestern zur Verfügung zu stellen, das frei von den geschilderten Nachteilen ist und das es gewährleistet, daß sich einerseits die verwendeten Katalysatoren im Reaktionsprodukt lösen und andererseits eine hohe Reaktivität aufweisen, so daß kürzere Reaktionszeiten, insbesondere bei gleicher Produktqualität möglich sind.

Die Aufgabe konnte gelöst werden durch Einsatz einer basischen Katalysatormischung aus Natrium- und Kaliumverbindungen in einem ausgewählten Mischungsverhältnis.

Ein Gegenstand der vorliegenden Erfindung betrifft ein Verfahren zur Herstellung von alkoxylierten Carbonsäureestern durch Umsetzung von Alkylenoxiden mit 2 bis 4 C-Atomen in Gegenwart eines basischen Katalysators, dadurch gekennzeichnet, daß man als basischen Katalysator eine Mischung von Natrium- und Kalium-Verbindungen aus der Gruppe der Hydroxide, Oxide, Carbonate, Alkoholate und Carboxylate in einem Gewichtsverhältnis von Natriumverbindungen zu Kalium-Verbindungen von 20 : 1 bis 1 : 20 einsetzt.

Während die Natrium- oder Kaliumverbindungen alleine als Katalysatoren nur eine geringe Aktivität aufweisen, so daß für die Alkoxylierung der Carbonsäureester technisch gesehen lange Reaktionszeiten in Kauf genommen werden müssen, wurde überraschenderweise gefunden, daß Mischungen von den beschriebenen Natrium- und Kaliumverbindungen in bestimmten Mischungsverhältnissen eine signifikant höhere Aktiviät aufweisen, so daß die gleiche Reaktion deutlich schneller verläuft.

### Carbonsäureester

Die als Einsatzstoffe für die Alkoxylierung in Betracht kommenden Carbonsäureester sind Ester von Carbonsäuren mit Monoalkoholen oder Ester von Carbonsäuren mit Polyolen. Bevorzugte Carbonsäuren sind Carbonsäuren mit 6 bis 22 Kohlenstoffatomen natürlicher oder synthetischer Herkunft, insbesondere geradkettige gesättigte und/oder ungesättigte Fettsäuren einschließlich technischer Gemische derselben, wie sie durch Fettspaltung aus tierischen und/oder pflanzlichen Fetten und Ölen zugänglich sind, zum Beispiel aus Kokosöl, Palmkemöl, Palmöl, Sojaöl, Sonnenblumenöl, Rüböl, Baumwollsaatöl, Fischöl, Rindertalg und Schweineschmalz. Beispiele für solche bevorzugten Carbonsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und/oder Erucasäure.

Geeignete Monoalkohole sind primäre Alkohole mit 1 bis 22 Kohlenstoffatomen, die gesättigt und/oder ungesättigt sein können. Beispiele für geeignete Monoalkohole sind Methanol, Ethanol, Propanol, Butanol, Pentanol sowie die Hydrierungsprodukte der oben genannten Carbonsäuren mit 6 bis 22 Kohlenstoffatomen. Insbesondere bevorzugt werden die Methylester der Carbonsäuren mit 6 bis 22 Kohlenstoffatomen.

Geeignete Polyole enthalten 2 bis 6 Hydroxylgruppen im Molekül und 2 bis 32 Kohlenstoffatome. Beispiele für geeignete Polyole sind Ethylenglykol, 1,2 Propylenglykol. 1,2-Butylenglykol, Neopentylglykol, Glycerin, Diglycerin, Triglycerin, Trimethylolpropan, Pentaerythrit sowie Sorbit. Sofern Carbonsäureester von Polyolen zum Einsatz kommen, können diese als Vollester oder als Partialester bzw. als Partialester enthaltende technische Estergemische, insbesondere in Form von Glyceriden vorliegen.

Im Sinne der vorliegenden Erfindung ist es besonders bevorzugt Methylester von Carbonsäuren mit 6 bis 22 Kohlenstoffatomen, insbesondere Methylester von Carbonsäuren mit 12 bis 18 Kohlenstoffatomen, als Ausgangsverbindung einzusetzen.

### Alkylenoxide

Zum Einsatz kommen als Alkylenoxide Ethylenoxid, Propylenoxid und/oder Butylenoxid, vorzugsweise Ethylenoxid und/oder Propylenoxid, insbesondere Ethylenoxid alleine.

### Katalysatoren

Als basische Katalysatoren wird eine Mischung von Natrium- Verbindungen und Kalium-Verbindungen aus der Gruppe der Hydroxide, Oxide, Carbonate, Alkoholate und Carboxylate eingesetzt. Als Alkoholate sind die entsprechenden Natrium- und Kalium-Verbindungen geeignet, die sich von Monoalkoholen mit 1 bis 6 Kohlenstoffatomen, vorzugsweise bis 4 Kohlenstoffatomen, ableiten, beispielsweise von Methanol, Ethanol, Propanol, n-Butanol oder t-Butanol. Als Carboxylate sind die entsprechenden Natrium- und Kaliumverbindungen geeignet, die sich von monofunktionellen Carbonsäuren mit 1 bis 22 Kohlenstoffatomen, vorzugsweise mit 2 bis 4 Kohlenstoffatomen, ableiten, beispielsweise von Essigsäure, Propionsäure, Buttersäure oder von den bereits im Zusammenhang mit den Carbonsäureestern beschriebenen Carbonsäuren mit 6 bis 22 Kohlenstoffatomen.

Bei den Katalysatormischungen können die Natrium- und Kalium-Verbindungen aus der gleichen Gruppe kommen, d.h. beides sind beispielsweise Hydroxide oder Alkoholate, oder aus verschiedenen Gruppen, d.h. eine Verbindung ist ein Alkoholat und die andere ein Carboxylat oder Hydroxid. Einer Ausführungsform der vorliegenden Erfindung entsprechend enthält die Katalysatormischung ein Natriumalkoholat, insbesondere Natriummethylat. Bevorzugt werden dementsprechend Katalysatormischungen, die bestehen aus Natriumalkoholaten, insbesondere Natriummethylat, und Kaliumhydroxid, Kaliumalkoholaten und/oder Kaliumcarboxylaten, insbesondere Kaliumhydroxid, Kaliummethylat, Kaliumbutylat ( von t-Butanol) und/oder Kaliumacetat. Einer anderen bevorzugten Ausführungsform der vorliegenden Erfindung entsprechend besteht die Katalysatormischung aus Natriumhydroxid und Kaliumhydroxid, Kaliumalkoholaten und/oder Kaliumcarboxylaten, insbesondere Kaliumhydroxid, Kaliummethylat, Kaliumbutylat ( von t-Butanol) und/oder Kaliumacetat.

Bei der erfindungsgemäß einzusetzenden Katalysatormischung beträgt das Mischungsverhältnis von Natrium-Verbindungen zu Kalium-Verbindungen 20 : 1 bis 1 : 20, vorzugsweise 10 : 1 bis 1 : 10 und insbesondere 1 : 1 bis 1 : 5. Im Sinne der Erfindung ist das Mischungsverhältnis als Gewichtsverhältnis der Natrium- Verbindung zur KaliumVerbindung definiert.

Die Katalysatormischung enthält ansonsten keine weiteren katalytisch aktiven Bestandteile.

Die Katalysatormischung wird vorzugsweise in Mengen von 0,2 bis 5 Gew.% - bezogen auf alkoxylierten Carbonsäureester als Reaktionsprodukt - eingesetzt. Höhere Gewichtsmengen sind zwar möglich, aber wirtschaftlich unrentabel. Niedrigere Gewichtsmengen sind zwar ebenfalls möglich, aber verlängern die Reaktionszeit.

### Alkoxylierung

Die Alkoxylierung kann in an sich bekannter Weise durchgeführt werden. Hierzu legt man üblicherweise den Carbonsäureester in einem Druckreaktor mit Rührer vor und setzt den homogenen Katalysator beispielsweise als Feststoff oder als Lösung, vorzugsweise in Wasser und/oder in Methanol, zu. Es hat sich als vorteilhaft erwiesen, den Autoklav vor der Reaktion gründlich mit Stickstoff zu spülen, um alle Spuren von Luftsauerstoff zu entfernen und etwa als Lösungsmittel eingesetztes Methanol durch Evakuieren zu entfernen. Danach wird der Druckbehälter aufgeheizt, wobei die Alkoxylierung vorzugsweise bei Temperaturen im Bereich von 100 bis 180 °C und insbesondere von 160 bis 180°C durchgeführt wird. Das Alkylenoxid wird über einen Heber in den Reaktor eingepreßt, wobei der autogene Druck bis auf etwa 5 bar ansteigen kann. Vorzugsweise werden pro Mol Carbonsäureester durchschnittlich 1 bis. 40 und insbesondere 5 bis 15 Mol Alkylenoxid, vorzugsweise Ethylenoxid eingesetzt. Mit anderen Worten liegt das Molverhältnis von Carbonsäureester zu Alkylenoxide im Bereich von 1 : 1 bis 1 : 40, vorzugsweise von 1 : 5 bis 1 : 15. Die Anlagerung des Alkylenoxids erfolgt dabei statistisch, d.h. bei der Insertion handelt es sich um keine hochselektive Reaktion bei der 1 Mol Fettsäurealkylester mit exakt n Mol Alkylenoxid reagiert. Vielmehr wird ein komplexes Gemisch unterschiedlich hoch alkoxylierter Ester erhalten. Das Ende der Reaktion ist daran zu erkennen, daß der Druck im Reaktor auf etwa 0,5 bar abfällt. Aus Sicherheitsgründen empfiehlt es sich, die Mischung noch weitere 30 Minuten zu rühren, ehe der Reaktor abgekühlt und entspannt wird. Der alkalische Katalysator kann, falls gewünscht, durch Zugabe von Säuren, beispielsweise Phosphorsäure, Essigsäure, Milchsäure oder dergleichen neutralisiert werden.

### Gewerbliche Anwendbarkeit

Nach dem erfindungsgemäßen Verfahren können in relativ kurzer Reaktionszeit von unter 4 Stunden die alkoxylierten Carbonsäureester hergestellt werden. Im Vergleich zu dem Einsatz von beispielsweise Natriummethylat alleine als basischer Katalysator bedeutet dies eine Halbierung der Reaktionszeit, insbesondere bei vergleichbarer Produktqualität.

Ein weiterer Gegenstand der vorliegenden Erfindung betrifft die Verwendung der nach dem erfindungsgemäßen Verfahren hergestellten alkoxylierten Carbonsäureester als Tensid zur Herstellung von Wasch-, Spül- und Reinigungsmitteln, insbesondere von Handgeschirrspülmitteln, Allweckreinigern, Bodenreinigem, Glasreinigern, automatischen Geschirrspülmitteln und flüssigen Waschmitteln.

Die alkoxylierten Carbonsäureester stellen nichtionische Tenside mit hoher Waschkraft dar und können daher in Kombination mit anderen anionischen, nichtionischen und/oder kationischen Tensiden in Wasch-, Spül- und Reinigungsmitteln eingesetzt werden, vorzugsweise in Mengen von 0,5 bis 30 Gew.%- bezogen auf Wasch-, Spül- und Reinigungsmitteln. In den entsprechenden Mitteln können weiterhin übliche Bestandteile in üblichen Mengen enthalten sein.

### Beispiele

### Allgemeine Herstellvorschrift.

In einem 1-l-Rührautoklaven wurden 290 g (1,34 Mol) Laurinsäuremethylester vorgelegt und mit der vorgesehenen Menge Katalysatormischung versetzt. Der Autoklav wurde verschlossen und dreimal abwechselnd mit Stickstoff gespült und 30 Minuten bei einer Temperatur von 100 °C evakuiert, um die Anwesenheit von Luftsauerstoff auszuschließen. Anschließend wurde die Reaktionsmischung unter Stickstoffabdeckung auf 165 bis 175°C erhitzt und portionsweise 710 g (16,13 Mol) Ethylenoxid eindosiert, wobei der autogene Druck zunächst bis auf 3,5 bar anstieg. Die Reaktion wurde fortgeführt, bis der Druck auf 0,5 bar abgesunken war. Nach weiteren 30 Minuten Nachrührzeit wurde der Druckreaktor abgekühlt und entspannt. Die Ergebnisse der Versuche sind in Tabelle 1 zusammengefaßt. Die Mengenangaben (Gew.%) bei den Katalysatoren beziehen sich auf das Endprodukt (ethoxylierter Laurinsäureester).

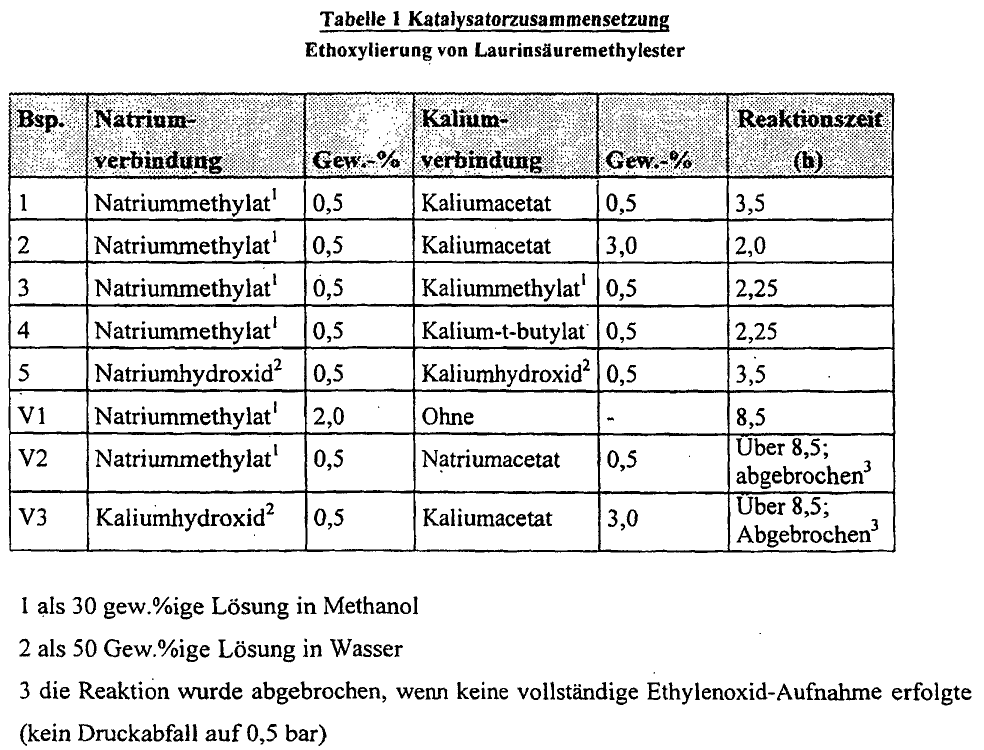

## Patentansprüche

1. Verfahren zur Herstellung von alkoxylierten Carbonsäureestern durch Umsetzung von Carbonsäureestern mit Alkylenoxiden mit 2 bis 4 C-Atomen in Gegenwart eines basischen Katalysators, **dadurch gekennzeichnet, daß** man als basischen Katalysator eine Mischung von Natrium- und Kalium-Verbindungen aus der Gruppe der Hydroxide, Oxide, Carbonate, Alkoholate und Carboxylate in einem Gewichtsverhältnis von Natriumverbindungen zu Kalium-Verbindungen von 20 : 1 bis 1 : 20 einsetzt.

2. Verfahren nach Anspruch 1, **dadurch gekennzeichnet, daß** man die Natrium- und Kalium-Verbindungen in einem Mischungsverhältnis von 10 : 1 bis 1 : 10, vorzugsweise 1 : 1 bis 1 : 5 einsetzt.

3. Verfahren nach einem der Ansprüche 1 und 2, **dadurch gekennzeichnet, daß** man Natriumalkoholate, vorzugsweise Natriummethylat, in Mischung mit Kaliumhydroxid, Kaliumalkoholaten und/oder Kaliumcarboxylaten einsetzt.

4. Verfahren nach einem der Ansprüche 1 bis 3, **dadurch gekennzeichnet, daß** man Natriumhydroxid in Mischung mit Kaliumhydroxid, Kaliumalkoholaten und/oder Kaliumcarboxylaten, vorzugsweise Kaliumhydroxid, Kaliummethylat, Kaliumbutylat und/oder Kaliumacetat, einsetzt.

5. Verfahren nach einem der Ansprüche 1 bis 4, **dadurch gekennzeichnet, daß** man die Katalysatormischung in Mengen von 0,2 bis 5 Gew.% - bezogen auf alkoxylierten Carbonsäureester - einsetzt.

6. Verfahren nach einem der Ansprüche 1 bis 5, **dadurch gekennzeichnet, daß** man als Carbonsäureester Ester von Carbonsäuren mit 6 bis 22 Kohlenstoffatomen und Monoalkoholen mit 1 bis 22 Kohlenstoffatomen oder mit Polyolen mit 2 bis 6 Hydroxylgruppen und 2 bis 32 Kohlenstoffatomen, insbesondere Methylester von Carbonsäuren mit 6 bis 22 Kohlenstoffatomen einsetzt.

7. Verfahren nach einem der Ansprüche 1 bis 6, **dadurch gekennzeichnet, daß** man als Alkylenoxide Ethylenoxid einsetzt.

8. Verfahren nach einem der Ansprüche 1 bis 7, **dadurch gekennzeichnet, daß** man die Carbonsäureester und Alkylenoxide in einem Molverhältnis von 1 : 1 bis 1 : 40 einsetzt.

9. Verfahren nach einem der Ansprüche 1 bis 8, **dadurch gekennzeichnet, daß** man die Umsetzung unter autogenem Druck bei Temperaturen im Bereich von 100 bis 180°C, vorzugsweise bei 160 bis 180°C durchführt.

## Claims

1. A process for the production of alkoxylated carboxylic acid esters by reacting carboxylic acid esters with alkylene oxides containing 2 to 4 carbon atoms in the presence of a basic catalyst, **characterized in that** a mixture of sodium and potassium compounds from the group of hydroxides, oxides, carbonates, alcoholates and carboxylates in a ratio by weight of sodium to potassium compounds of 20:1 to 1:20 is used as the basic catalyst.

2. A process as claimed in claim 1, **characterized in that** the sodium and potassium compounds are used in a mixing ratio of 10:1 to 1:10 and preferably 1:1 to 1:5.

3. A process as claimed in claim 1 or 2, **characterized in that** sodium alcoholates, preferably sodium methylate, are used in admixture with potassium hydroxide, potassium alcoholates and/or potassium carboxylates.

4. A process as claimed in any of claims 1 to 3, **characterized in that** sodium hydroxide is used in admixture with potassium hydroxide, potassium alcoholates and/or potassium carboxylates, preferably potassium hydroxide, potassium methylate, potassium butylate and/or potassium acetate.

5. A process as claimed in any of claims 1 to 4, **characterized in that** the catalyst mixture is used in quantities of 0.2 to 5% by weight, based on alkoxylated carboxylic acid ester.

6. A process as claimed in any of claims 1 to 5, **characterized in that** esters of C₆₋₂₂ carboxylic acids and C₁₋₂₂ monoalcohols or with polyols containing 2 to 6 hydroxyl groups and 2 to 32 carbon atoms, more particularly methyl esters of C₆₋₂₂ carboxylic acids, are used as the carboxylic acid esters.

7. A process as claimed in any of claims 1 to 6, **characterized in that** ethylene oxide is used as the alkylene oxide.

8. A process as claimed in any of claims 1 to 7, **characterized in that** the carboxylic acid esters and alkylene oxides are used in a molar ratio of 1:1 to 1:40.

9. A process as claimed in any of claims 1 to 8, **characterized in that** the reaction is carried out under autogenous pressure at temperatures in the range from 100 to 180°C and preferably at temperatures in the range from 160 to 180°C.

## Revendications

1. Procédé pour la préparation d'esters d'acides carboxyliques alcoxylés par réaction d'esters d'acides carboxyliques avec des oxydes d'alkylène comportant 2 à 4 atomes de carbone en présence d'un catalyseur basique,
**caractérisé en ce que**
comme catalyseur basique, on utilise un mélange de composés du sodium et du potassium appartenant au groupe des hydroxydes, des oxydes, des carbonates, des alcoolates et des carboxylates, dans un rapport pondéral des composés du sodium aux composés du potassium de 20 : 1 à 1 : 20.

2. Procédé selon la revendication 1,
**caractérisé en ce que**
les composés du sodium et du potassium sont utilisés dans un rapport de 10 : 1 à 1 : 10, de préférence 1 : 1 à 1 : 5.

3. Procédé selon l'une quelconque des revendications 1 et 2,
**caractérisé en ce que**
comme alcoolates de sodium, on utilise de préférence le méthylate de sodium, en mélange avec de l'hydroxyde de potassium, des alcoolates de potassium et/ou des carboxylates de potassium.

4. Procédé selon l'une quelconque des revendications 1 à 3,
**caractérisé en ce que**
l'hydroxyde de sodium est utilisé en mélange avec de l'hydroxyde de potassium, des alcoolates de potassium, et/ou des carboxylates de potassium, de préférence l'hydroxyde de potassium, le méthylate de potassium, le butylate de potassium et/ou l'acétate de potassium.

5. Procédé selon l'une quelconque des revendications 1 à 4,
**caractérisé en ce que**
le mélange catalyseur est utilisé dans les proportions de 0,2 à 5 % en poids - par rapport à l'ester d'acide carboxylique alcoxylé.

6. Procédé selon l'une quelconque des revendications 1 à 5,
**caractérisé en ce que**
comme esters d'acides carboxyliques, on utilise des esters d'acides carboxyliques comportant 6 à 22 atomes de carbone avec des monoalcools comportant 1 à 22 atomes de carbone ou avec des polyols comportant 2 à 6 groupes hydroxyle et 2 à 32 atomes de carbone, en particulier des esters méthyliques d'acides carboxyliques comportant 6 à 22 atomes de carbone.

7. Procédé selon l'une quelconque des revendications 1 à 6,
**caractérisé en ce que**
comme oxyde d'alkylène, on utilise l'oxyde d'éthylène.

8. Procédé selon l'une quelconque des revendications 1 à 7,
**caractérisé en ce que**
les esters d'acides carboxyliques et les oxydes d'alkylène sont utilisés dans un rapport molaire de 1 : 1 à 1 : 40.

9. Procédé selon l'une quelconque des revendications 1 à 8,
**caractérisé en ce que**
la réaction sous pression autogène s'effectue à des températures se situant dans une plage de 100 à 180°C, de préférence dans une plage de 160 à 180 °C.
